# EUROPEAN PATENT SPECIFICATION

(11) **EP 0 429 522 B1**
(45) Date of publication and mention of the grant of the patent: **26.06.1996**
(21) Application number: 89909685.3
(22) Date of filing: 18.08.1989
(51) Int. Cl.: A61K 31/725, A61K 31/71, C07H 11/04

(54) **PHOSPHOSUGAR-BASED ANTI-INFLAMMATORY AND/OR IMMUNOSUPPRESSIVE DRUGS**
AUF PHOSPHOZUCKER BASIERENDE ANTIENTZÜNDLICHE UND/ODER IMMUNITÄTSUNTERDRÜCKENDE ARZNEIMITTEL
MEDICAMENTS ANTI-INFLAMMATOIRES ET/OU IMMUNOSUPPRESSEURS A BASE DE PHOSPHOGLUCIDE

(30) Priority: 19.08.1988 AU 9942/88
(43) Date of publication of application: 05.06.1991
(73) Proprietor: THE AUSTRALIAN NATIONAL UNIVERSITY, Acton, Australian Capital Territory 2601 (AU)
(72) Inventor: PARISH, Christopher, Richard, Campbell, ACT 2601 (AU); COWDEN, William, Butler, Crozier Circuit Kambah, ACT 2902 (AU); WILLENBORG, David, Otto, Sterling, ACT 2611 (AU)
(74) Representative: Woods, Geoffrey Corlett
(86) International application number: AU8900350
(87) International publication number: WO9001938

(56) References cited:
- EP-A- 0 158 879
- EP-A- 0 194 710
- AU-A- 2 278 588
- AU-A- 5 931 386
- DE-A- 0 334 250
- GB-A- 2 144 332
- GB-A- 2 185 398
- US-A- 4 122 179
- US-A- 4 448 771
- US-A- 4 703 040
- US-A- 4 761 402
- PATENT ABSTRACTS OF JAPAN, vol. 12, no. 365 (C-532)[3212], 29th September 1988;& JP-A-63 119 427 (KAO) 24-05-1988
- PATENT ABSTRACTS OF JAPAN, vol. 7, no. 225 (C-189)[1370], 6th October 1983;& JP-A-58 121 216 (GRELAN SEIYAKU) 19-07-1983
- THE JOURNAL OF IMMUNOLOGY, vol. 134, no. 1, January 1985, pages 65-69; H.-D.HAUBECK et al.: "Natural killer cell-mediated cytotoxicity does not depend onrecognition of mannose 6-phosphate residues"
- The Merck Index, Tenth Edition, issued 1983 (RAHWAY, N.J., USA) see Monographs4151, 4152, 4322 and 4323 at pages 609 and 639
- PATENT ABSTRACTS OF JAPAN, C144, page 7, JP,A, 57-163491 (Nisshin Seifon K.K.)7 October 1982 (07.10.82)
- PATENT ABSTRACTS OF JAPAN, C553, page 48, JP,A, 63-198629 (Kao Corp) 17 August1988 (17.08.88)
- WEST, C.M. "Molecular and Cellular Biochemistry", Volume 72, published 1986, byMartinus Nijhoff (Boston) see pages 3 to 20, Especially page 8
- Journal of Biological Chemistry, Volume 258, No 5, issued 10 March 1985, AJITVARKI et al "The Spectrum of Anionic Oligosaccharides..." see pages 2808 to2814
- Journal of Biological Chemistry, Volume 257, No. 17, issued 10 September 1982.
- H.DAVID FISCHER et al, "Binding of Phosphorylated Oligosaccharides toImmobilized Phosphomannosyl Receptors" see pages 9938 to 9943
- Archives of Biochemistry and Biophysics Volume 214, No. 2, issued 2 April 1982(Academic Press, Inc.), ANTON W. STEINER et al, "Assay and Purification of aSolobilized Membrane Receptor that Binds the Lysosomal Enzyme -L-Iduronidase",see pages 681 to 687
- Annual Review of Biochemistry, volume 55 issued 1986 (Annual Reviews Inc)KURTVON FIGURA et al, "Lysosomal Enzymes and their Receptors", pages 167 to 193

## Description

This invention relates to phosphosugars and phosphosugar containing compounds that possess anti-inflammatory and/or immunosuppressive activity, and in particular it relates to the use of these compounds as anti-inflammatory and/or immunosuppressive agents in animals and man.

The lysosomes of cells contain a wide range of degradative enzymes which play a central role in the entry of leukocytes into inflammatory sites. Lysosomal enzymes, produced in the rough endoplasmic reticulum, undergo glycosylation followed by a number of 'trimming' and phosphorylation reactions resulting in oligosaccharides rich in mannose-6-phosphate residues (1-3). These mannose-6-phosphate residues are specific recognition markers of lysosomal enzymes (3). It is this marker on the enzymes that is recognized by a mannose phosphate receptor (MPR) which mediates transport of lysomsomal enzymes to lysosomes. This receptor functions not only in internal transport of lysosomal enzymes but is also important in their secretory pathway and their expression on cell surfaces (1). Receptor-lysosomal enzyme interactions have been extensively studied (4-6) and shown to be inhibited by exogenous mannose-6-phosphate. Work leading to the present invention has been based on the hypothesis that mannose-6-phosphate and related phosphosugar structures might act as anti-inflammatory agents in vivo, possibly by depleting leukocytes of their lysosomal enzymes although this has not been shown previously.

As a result of these investigations, it has now been discovered that certain phosphosugars, notably mannose-6-phosphate and fructose-1-phosphate, are in fact effective anti-inflammatory agents, continuous infusion of the sugars inhibiting experimental allergic encephalomyelitis (EAE), an animal inflammatory disease of the central nervous system resembling multiple sclerosis in humans. Polysaccharides containing D-mannose with phosphate residues have also been found to inhibit EAE.

Phosphosugars, particularly mannose-6-phosphate, have also been found to exhibit an anti-inflammatory effect on passively induced adjuvant arthritis. Adjuvant-induced arthritis in the rat shares a number of features with arthritis in humans, viz. the presence of a proliferative synovitis and subcutaneous nodules, swelling of extremities, and ultimately cartilage and bone erosion. This animal model has been extensively used for detection of anti-inflammatory and immunosuppressive drugs.

Finally, phosphosugars have been found to be effective as an immunosuppressant in preliminary experiments, particularly in controlling the delayed hypersensitivity reaction.

The present invention therefore provides the use of at least one phosphosugar selected from the group consisting of mannose-6-phosphate, fructose-1-phosphate, fructose-6-phosphate, galactose-6-phosphate, and fructose-1,6-diphosphate, or derivative thereof, or a phosphosugar-containing oligosaccharide or polysaccharide containing phosphomannose residues or a derivative thereof, which is an antagonist or competitive inhibitor of the natural ligand of mannose phosphate receptors, for the manufacture of a medicament for anti-inflammatory and/or immunosuppressive treatment of an animal or human patient. The medicament may be in the form of a pharmaceutical or veterinary composition which comprises at least one phosphosugar as aforesaid, together with an acceptable pharmaceutical or veterinary carrier or diluent therefor.

Phosphosugars and phosphosugar-containing oligosaccharides or polysaccharides which may be used in accordance with the present invention comprise both naturally occurring and synthetic compounds containing or comprising phosphosugar residues, that is, sugar residues bearing at least one phosphate moiety. Particularly useful phosphosugars include phosphomannoses, phosphofructoses, phosphogalactoses and phosphoglucoses, while particularly useful oligosaccharides or polysaccharides include polysaccharides containing phosphomannose residues. Presently preferred phosphosugars include mannose-6- phosphate and fructose-1-phosphate. Preferred phosphosugar derivatives are the esters including acetate esters, particularly the 1,2,3,4-tetra-O-acetyl derivative of mannose-6-phosphate.

Whilst it is not intended that the present invention should be restricted in any way by a theoretical explanation of the mode of action of the phosphosugars in accordance with the invention, it is presently believed that these active compounds may exert their own anti-inflammatory effect, by acting as antagonists or competitive inhibitors of the natural ligand of mannose phosphate receptors (MPR) on cells. Accordingly, the active phosphosugars or phosphosugar-containing oligosaccharides or polysaccharides may include any such compounds which are effective antagonists or competitive inhibitors of the natural ligand of the MPR.

The active anti-inflammatory and/or immunosuppressive agents in accordance with the present invention may be used to treat inflammatory diseases or conditions such as multiple sclerosis and rheumatoid arthritis, as well as in the treatment of the inflammatory process associated with the rejection of organ transplants (since massive mononuclear cell infiltrates are usually associated with acute graft rejection). These active agents may be used alone, in combination with one or more other phosphosugars, or in combination with other known anti-inflammatory or immunosuppressive agents. In particular, compositions of phosphosugars and sulphated polysaccharides with heparanase-inhibitory activity may act synergistically and represent a formulation with potent anti-inflammatory activity. The anti-inflammatory activity of these sulphated polysaccharides is disclosed in detail in International Patent Application No. PCT/AU88/00017.

The anti-inflammatory and/or immunosuppressive activity and use of the phosphosugars in accordance with the present invention is demonstrated in the following Example.

### EXAMPLE 1 Inhibition of EAE.

In this Example, a number of phosphosugars and one phospho-polysaccharide were tested for their ability to inhibit development of EAE in rats. (All phosphosugars tested are commercially available and were obtained from Sigma Chemical Co., St. Louis, MO, U.S.A.). Experimental details are included in the footnotes to the Tables setting out the test results.

Table I presents data from an EAE experiment in rats where mannose-6-phosphate, administered to animals via osmotic pumps, totally inhibited development of disease. The data presented in Table II demonstrates that a four fold reduction in the mannose-6-phosphate dose (40 mg/rat/week to 10 mg/rat/week) still resulted in a substantial reduction in disease severity, i.e. the lowest dose of phosphosugar reduced disease severity to 37.7% that of control animals.

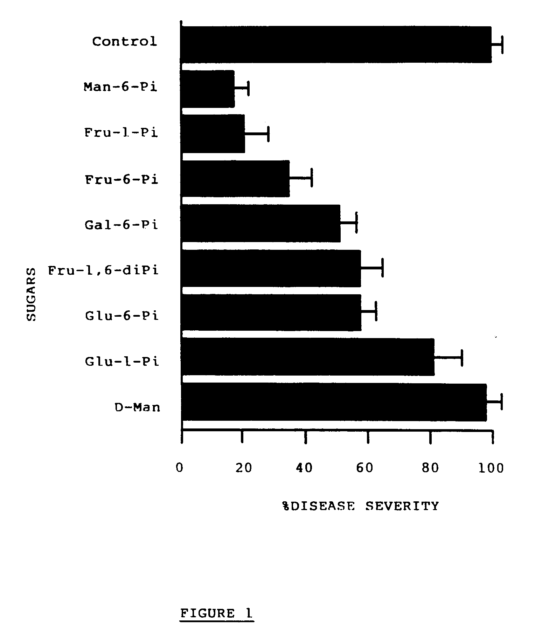

Analysis of phosphosugar specificity revealed (Table III) that fructose-1-phosphate was as effective as mannose-6-phosphate at inhibiting disease. Fructose-6-phosphate was also a comparatively effective inhibitor of EAE, whereas galactose-6-phosphate, glucose-6-phosphate and fructose-1,6-diphosphate were partially inhibitory. Glucose-1-phosphate and D-mannose apparently had little or no effect on disease progression. These results are displayed graphically in Figure 1. Such phosphosugar specificity closely resembles the monosaccharide specificity of the mannose-6-phosphate receptors on cells (1).

In two separate experiments (Table IV) administration of the D-mannose polysaccharide (mannan) from Saccharomyces cerevisiae, which contains phosphate moieties, totally inhibited EAE, indicating that phosphomannans can inhibit disease.

Histological examination of central nervous system (CNS) tissue from untreated animals with EAE and EAE animals which had been treated with either mannose-6-phosphate or mannan containing phosphate moieties, (Table V) revealed that both treatments dramatically inhibited development of CNS lesions. No lesions were detected in mannose-6-phosphate treated animals and a small number of lesions, compared with controls, in mannan treated rats. Such data are consistent with the view that the sugars are inhibiting entry of leukocytes into the CNS.

The first data column in the Tables refers to the number of animals in each group which showed any clinical signs of EAE during the entire course of the experiment. Thus, although 7/10 animals treated with mannose-6-phosphate developed some clinical signs of disease (Table III) the severity of these disease symptoms was extremely mild compared with untreated animals, i.e., <10% disease severity of controls when clinical scores and duration of disease are examined. In this sense, the mannose-6-phosphate data in Tables I and III are almost identical. Similarly, the estimation of disease severity can be used to rank the anti-inflammatory activity of phosphosugars which only partially inhibit disease, e.g., glucose-6-phosphate and fructose-1,6-diphosphate.

**Table I**

| Effect of Mannose-6-Phosphate on Adoptively Transferred EAE | | | | |
|---|---|---|---|---|
| Treatment | No. with EAE/Total | Mean Day Onset | Mean Clinical Score | Mean Length Disease (days) |
| Control | 8/8 | 5.2 | 3.0 | 3.5 |
| Mannose-6-phosphate | 0/8 | 0 | 0 | 0 |
| Legend to Table I: EAE induced in Lewis rats with 30 x 10⁶ ConA activated EAE effector cells. Miniosomotic pumps containing phosphosugar were implanted subcutaneously on day 3 after cell transfer. Dose was 40 mg/rat delivered over a 7 day period by 2.0 ml pumps. Clinical EAE was graded according to the following scheme: 0, asymptomatic; 1, flaccid distal half of tail; 2, entire tail flaccid; 3, ataxia, difficulty in righting; 4, hind limb weakness; and 5, hind limb paralysis. | | | | |

**Table II**

| Effect of Mannose-6-Phosphate Dose on Adoptively Transferred EAE | | | | | | |
|---|---|---|---|---|---|---|
| Treatment | Dose (mg) | No. with EAE/total | Mean Day Onset | Mean Clinical Score | Mean Length Disease | Disease Severity (%Control) |
| Control | - | 4/4 | 4.5 | 3.5 | 4.5 | 100% |
| Mannose-6-Phosphate | 40 | 1/3 | 5.0 | 0.3 | 0.7 | 1.7% |
| Mannose-6-Phosphate | 20 | 4/4 | 5.0 | 1.5 | 3.0 | 28.6% |
| Mannose-6-Phosphate | 10 | 4/4 | 5.0 | 1.8 | 3.3 | 37.7% |
| Legend to Table II: Experimental details as in Table I. Mannose-6-phosphate dose represents amount of phosphosugar delivered to rats over a 7 day period via mino-osmotic pumps. "Disease Severity" represents product of mean clinical score and mean length disease. | | | | | | |

**Table III**

| Phosphosugar Specificity of EAE Inhibition | | | | | |
|---|---|---|---|---|---|
| Treatment | No.with EAE/Total | Mean Day Onset | Mean Clinical Score | Mean Length Disease (days) | Disease Severity (%Control) |
| Control | 9/9 | 5.0 | 3.6 | 4.2 | 100% |
| Mannose-6-phosphate | 7/10 | 6.0 | 0.9 | 1.5 | 8.9% |
| Fructose-1-phosphate | 3/5 | 5.5 | 1.2 | 1.6 | 12.6% |
| Fructose-6-phosphate | 4/5 | 6.0 | 1.6 | 2.4 | 25.4% |
| Galactose-6-phosphate | 5/5 | 5.2 | 2.0 | 3.0 | 40.5% |
| Glucose-6-phosphate | 5/5 | 5.4 | 2.0 | 3.8 | 50.3% |
| Fructose-1,6-diphosphate | 5/5 | 5.4 | 2.4 | 3.4 | 54.0% |
| Glucose-1-phosphate | 5/5 | 5.2 | 3.0 | 3.8 | 75.5% |
| D-mannose | 5/5 | 5.2 | 2.9 | 4.4 | 84.5% |
| Legend to Table III: Experimental details as in Table I. "Disease Severity" represents product of mean clinical score and mean length disease. | | | | | |

**Table IV**

| Inhibition of Adoptively Transferred EAE by Yeast Mannan | | | | |
|---|---|---|---|---|
| Treatment | No. with EAE/Total | Mean Day Onset | Mean Clinical Score | Mean Length Disease (days) |
| | | | | |

| Expt. 1 | | | | |
|---|---|---|---|---|
| Control | 5/5 | 4.8 | 3.5 | 4.0 |
| Yeast mannan | 0/6 | 0 | 0 | 0 |

| Expt. 2 | | | | |
|---|---|---|---|---|
| Control | 4/4 | 5.0 | 3.1 | 3.7 |
| Yeast mannan | 0/4 | 0 | 0 | 0 |
| Legend to Table IV: Yeast mannan from Saccharomyces cerevisiae (Baker's yeast). Experimental details as in Table I. | | | | |

**Table V**

| Histological analysis of EAE Inhibition in Rats Receiving Mannose-6-Phosphate and Mannan | | | |
|---|---|---|---|
| Treatment | No. Sections scanned | No.Lesions | Lesions/section |
| | | | |

| Expt. 1 | | | |
|---|---|---|---|
| Control 1 | 10 | 110 | 11.0 |
| Control 2 | 8 | 206 | 25.7 |
| Mannose-6-phosphate 1 | 18 | 0 | 0 |
| Mannose-6-phosphate 2 | 15 | 0 | 0 |

| Expt. 2 | | | |
|---|---|---|---|
| Control 1 | 15 | 284 | 19.0 |
| Control 2 | 12 | 303 | 25.0 |
| Yeast mannan 1 | 18 | 20 | 1.1 |
| Yeast mannan 2 | 15 | 92 | 6.7 |
| Legend to Table V: Rats were killed 9 days after cell transfer and sections of the lower thoracic-upper lumbar spinal cord examined for inflammatory lesions. Animals treated as in Table I. | | | |

### EXAMPLE 2 Inhibition of EAE

In further experiments using the EAE model of Example 1, other mannose phosphate-containing compounds were used, including PPME and a pentasaccharide.

PPME is the purified high molecular weight, acid-resistant fragment, (polysaccharide core fraction) of the isolated exocellular phosphomannan produced by Pichia holstii (Hansenula holstii) as described by Bretthauer et.al. (7), that contains mannose phosphate residues.

The pentasaccharide is an isolated monophosphomannopentaose fragment, 6-phospho-mannose-α(1-3)-{mannose-α-(1-3)}₂-mannose-α-(1-2)-mannose, of the exocellular phosphomannan produced by Pichia holstii (Hansenula holstii) described by Bretthauer et.al. (7).

In these experiments, details of which were as in Table I, the number of cells transferred was 25x10⁶/rat, while the dose of compound administered was 10mg/rat delivered over a 7 day period by mini-osmotic pumps, commencing on day 3 after cell transfer. The results are set out in Table VI.

**Table VI**

| | Control | PPME | Pentasaccharide |
|---|---|---|---|
| EAE/Total | 5/5 | 3/5 | 1/5 |

### EXAMPLE 3 Suppression of Passive Adjuvant Arthritis

(DA x Lew)Fl rats were immunized with M.butyricum in light mineral oil given in each foot. Ten days later spleens were removed and incubated as single cell suspension tissue culture medium in + 5µg/ml ConA for 75 hrs. Cells were harvested, washed and transferred i.v. at 65 x 10⁶ cells/rat into (DA x Lew)F recipients.

Treated rats were implanted on the day they received cells with miniosmotic pumps which delivered 6mg/rat/day of mannose-6- phosphate for 14 days. Control rats were sham operated. The results are shown in Table VII as % of pre-cell injection foot size. {Average for group; n=4 (mannose-6-phosphate); n=6 (control)}.

**Table VII**

| Day | Mannose-6-Phosphate | Control |
|---|---|---|
| 4 | 97.3% | 106.4% |
| 6 | 105.8% | 129.7% |
| 7 | 102.8% | 149.7% |
| 9 | 108.4% | 148.5% |
| 11 | 107.6% | 184.2% |
| 14 | 117.4% | 220.1% |

### EXAMPLE 4 Effect on Delayed-Type Hypersensitivity (DTH)

C57Bl mice were sensitised by i.v. injection 10⁵ of washed sheep red blood cells. 5 days later they were challenged in the right hind footpad with SRBC. Each mouse was given a 0.25ml injection i/p at the same time of either saline, mannose-6-phosphate or the 1,2,3,4-tetraacetate of mannose-6-phosphate and all injections were repeated a further 6 times at approx. 3½ hour intervals. The dose in each injection of mannose-6- phosphate was 0.15mg and of 1,2,3,4-tetra-O-acetyl derivative of mannose-6-phosphate was also 0.15mg. At 24 hours after challenge the DTH swelling was measured. Mannose-6-phospate reduced the swelling by 52.5%, and the 1,2,3,4-tetra-O-acetyl derivative of mannose-6- phosphate by 91.5%, as compared with the saline controls.

### REFERENCES

1. von Figura, K. and Hasilik, A. (1986). Ann. Rev. Biochem. 55 : 167.
2. West, C.M. (1986). Mol. Cell. Biochem. 72 : 3.
3. Hickman, S. and Neufeld, E.F. (1972). Biochem. Biophys. Res. Comm. 49 : 992.
4. Varki, A. and Kornfeld, S. (1983). J.Biol. Chem. 258 : 2808.
5. Fischer, H.D., Creek, K.E. and Sly, W.S. (1982). J. Biol. Chem. 257 : 9938.
6. Steiner, A.W. and Rome, L.H. (1982). Arch. Biochem. Biophys. 214 : 681.
7. Bretthauer, R.K., Kaczorowski, G.J. and Weise, M.J., (1973), Biochemistry 12(7) : 1251.

## Claims

1. Use of at least one phosphosugar selected from the group consisting of mannose-6-phosphate, fructose-1-phosphate, fructose-6-phosphate, galactose-6-phosphate, and fructose-1,6-diphosphate, or derivative thereof, or a phosphosugar-containing oligosaccharide or polysaccharide containing phosphomannose residues or a derivative thereof, which is an antagonist or competitive inhibitor of the natural ligand of mannose phosphate receptors, for the manufacture of a medicament for anti-inflammatory and/or immunosuppressive treatment of an animal or human patient.

2. Use according to claim 1 wherein the phosphosugar is mannose-6-phosphate.

3. Use according to claim 1 wherein the phosphosugar is fructose-1-phosphate.

4. Use according to claim 1 wherein the phosphosugar is fructose-6-phosphate.

5. Use according to claim 1 wherein the phosphosugar derivative comprises an acetate or other ester thereof.

6. Use according to claim 5 wherein the phosphosugar ester is the 1,2,3,4-tetra-O-acetyl-derivative of mannose-6-phosphate.

7. Use according to claim 1 wherein the oligosaccharide, or polysaccharide is the D-mannose polysaccharide (mannan) from Saccharomyces cerevisiae.

8. Use according to claim 1 wherein the oligosaccharide or polysaccharide is the purified high molecular weight, acid-resistant fragment (polysaccharide core fraction) of the exocellular phosphomannan produced by Pichia holstii (Hansenula holstii), or an oligosaccharide fragment derived therefrom.

9. Use according to claim 8 wherein the oligosaccharide fragment is the monophosphomannopentaose fragment, 6-phospho-mannose-α(1-3)-{mannose-α-(1-3)}₂-mannose-α-(1-2)-mannose.

10. Use according to any one of claims 1 to 9 wherein the treatment comprises treatment of inflammatory disease of the central nervous system.

11. Use according to any one of claims 1 to 9 wherein the treatment comprises treatment of arthritis.

12. Use according to any one of claims 1 to 9 wherein the treatment comprises treatment to control the delayed-type hypersensitivity reaction.

## Patentansprüche

1. Verwendung mindestens eines Phosphorzuckers, ausgewählt aus der Gruppe Mannose-6-phosphat, Fructose-1-phosphat, Fructose-6-phosphat, Galactose-6-phosphat und Fructose-1,6-diphosphat, oder eines Derivats hiervon oder eines phosphorzuckerhaltigen Oligosaccharids oder Polysaccharids mit Phosphomannoseresten oder eines Derivats hiervon, das einen Antagonisten oder konkurrierenden Inhibitor des natürlichen Liganden von Mannosephosphatrezeptoren darstellt, zur Herstellung eines Medikaments zur Entzündungshemm- und/oder Immunsuppressivbehandlung eines tierischen oder menschlichen Patienten.

2. Verwendung nach Anspruch 1, wobei der Phosphorzucker aus Mannose-6-phosphat besteht.

3. Verwendung nach Anspruch 1, wobei der Phosphorzucker aus Fructose-1-phosphat besteht.

4. Verwendung nach Anspruch 1, wobei der Phosphorzucker aus Fructose-6-phosphat besteht.

5. Verwendung nach Anspruch 1, wobei das Phosphorzuckerderivat aus einem Acetat oder sonstigen Ester desselben besteht.

6. Verwendung nach Anspruch 5, wobei der Phosphorzuckerester aus dem 1,2,3,4-Tetra-O-acetylderivat von Mannose-6-phosphat besteht.

7. Verwendung nach Anspruch 1, wobei das Oligosaccharid oder Polysaccharid aus dem D-Mannosepolysaccharid (Mannan) von Saccharomyces cerevisiae besteht.

8. Verwendung nach Anspruch 1, wobei das Oligosaccharid oder Polysaccharid aus dem gereinigten, hochmolekularen säureresistenten Fragment (Polysaccharidkernfraktion) des durch Pichia holstii (Hansenula holstii) produzierten exozellulären Phosphomannans oder einem davon herrührenden Oligosaccharidfragment besteht.

9. Verwendung nach Anspruch 8, wobei das Oligosaccharidfragment aus dem Monophosphormannopentaose-Fragment, 6-Phospho-mannose-α(1-3)-{mannose-α-(1-3)}₂-mannose-α-(1-2)-mannose besteht.

10. Verwendung nach einem der Ansprüche 1 bis 9, wobei die Behandlung die Behandlung einer entzündlichen Erkrankung des Zentralnervensystems umfaßt.

11. Verwendung nach einem der Ansprüche 1 bis 9, wobei die Behandlung eine Arthritisbehandlung umfaßt.

12. Verwendung nach einem der Ansprüche 1 bis 9, wobei die Behandlung die Behandlung oder Steuerung der verzögerten Überempfindlichkeitsreaktion umfaßt.

## Revendications

1. Utilisation d'au moins un phosphosucre choisi parmi les mannose-6-phosphate, fructose-1-phosphate, fructose-6-phosphate, galactose-6-phosphate et fructose-1,6-diphosphate ou leurs dérivés, ou un oligosaccharide contenant un phosphosucre ou un polysaccharide contenant des restes phosphomannose ou un dérivé de celui-ci, qui est un antagoniste ou un inhibiteur compétitif du ligand naturel des récepteurs mannose-phosphate, pour la fabrication d'un médicament pour un traitement anti-inflammatoire et/ou immuno-suppresseur d'un patient humain ou animal.

2. Utilisation selon la revendication 1, dans laquelle le phosphosucre est le mannose-6-phosphate.

3. Utilisation selon la revendication 1, dans laquelle le phosphosucre est le fructose-1-phosphate.

4. Utilisation selon la revendication 1, dans laquelle le phosphosucre est le fructose-6-phosphate.

5. Utilisation selon la revendication 1, dans laquelle le dérivé de phosphosucre comprend un acétate ou un autre ester de celui-ci.

6. Utilisation selon la revendication 5, dans laquelle l'ester de phosphosucre est le dérivé 1,2,3,4-tetra-O-acétyle de mannose-6-phosphate.

7. Utilisation selon la revendication 1, dans laquelle l'oligosaccharide ou le polysaccharide est le polysaccharide D-mannose (mannane) issu de Saccharomyces cerevisiae.

8. Utilisation selon la revendication 1, dans laquelle l'oligosaccharide ou le polysaccharide est le fragment purifié à haute masse moléculaire et résistant aux acides (fraction du noyau polysaccharide) du phosphomannane exocellulaire produit par Pichia holstii (Hansenula holstii) ou un fragment d'oligosaccharide dérivé.

9. Utilisation selon la revendication 8, dans laquelle le fragment d'oligosaccharide est le fragment monophosphomannopentaose, 6-phospho-mannose-alpha(1-3)-(mannose-alpha-(1-3)₂-mannose-alpha-(1-2)-mannose.

10. Utilisation selon l'une quelconque des revendications 1 à 9, dans laquelle le traitement comprend le traitement d'une maladie inflammatoire du système nerveux central.

11. Utilisation selon l'une quelconque des revendications 1 à 9, dans laquelle le traitement comprend le traitement de l'arthrite.

12. Utilisation selon l'une quelconque des revendications 1 à 9, dans laquelle le traitement comprend un traitement de régulation de la réaction d'hypersensibilité du type retard.
